# EUROPEAN PATENT APPLICATION

(11) **EP 2 392 351 A1**
(43) Date of publication of application: **07.12.2011**
(21) Application number: 10735603.2
(22) Date of filing: 21.01.2010
(51) Int. Cl.: A61K 39/395, A61K 35/76, A61K 48/00, A61P 31/18, C07K 16/28, C12N 15/00

(54) **HIV REPLICATION INHIBITOR, AND USE THEREOF**

(30) Priority: 28.01.2009 JP 2009017340
(71) Applicant: Tokai University Educational System, Tokyo 151-0063 (JP)
(72) Inventor: TAKEKOSHI, Masataka, Isehara-shi Kanagawa 259-1193 (JP); TAKEKOSHI, Fumiko, Isehara-shi Kanagawa 259-1193 (JP)
(74) Representative: Blum, Erwin
(86) International application number: PCT/JP2010/000323
(87) International publication number: WO 2010/087131

(57) **Abstract**

It is an object of the present invention to provide an agent for suppressing the replication of HIV, which comprises a human CD4-recognizing antibody. The object is solved by an agent for suppressing the replication of human immunodeficiency virus, which comprises, as an active ingredient, an antibody described in (1) below:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing.

## Description

### Technical Field

The present invention relates to an agent for suppressing the replication of human immunodeficiency virus (HIV), which comprises, as an active ingredient, an antibody that recognizes human CD4 or a nucleic acid comprising nucleotide sequences encoding the antibody. Moreover, the present invention also relates to a pharmaceutical agent for preventing and/or treating acquired immune deficiency syndrome (AIDS), which comprises, as an active ingredient, the above-described agent for suppressing the replication of HIV.

### Background Art

It is very important to understand a mechanism for generating a self-recognizing antibody and natural tolerance *in vivo,* not only in the field of autoimmune disease but also in the field of development of AIDS vaccine. One reason is that several strong neutralizing antibodies (2F5, 4E10, b12, 2G12, etc.) directed against human immunodeficiency virus type 1 (HIV-1) show autoreactivity (see Non Patent Documents 1 and 2).

These antibodies showing broad neutralizing activity are rarely detected in people infected with HIV-1, and it has not been successful in enhancing the titers of these antibodies by immunization. One reason may be the cross-reactivity of a strong HIV-1 neutralizing antibody with an autoantigen. On the other hand, an autoreactive antibody is detected even in healthy people. Generation of a self-recognizing antibody and its pathophysiological role in healthy people have not fully been known.

Human CD4 is a T cell marker, which has been known to function as a receptor of HIV-1. To date, it has been reported that a human autoreactive CD4 antibody is detected in people infected with HIV-1 (approximately 13%) and in people exposed to HIV-1 who tested negative in a serum reaction (34%), and that some healthy people (approximately 0.6%) test positive to a human anti-CD4 antibody (see Non Patent Documents 3 to 5). Moreover, several types of mouse anti-CD4 monoclonal antibodies have been known to inhibit the replication of HIV-1 *in vitro* and *in vivo* (see Non Patent Documents 6 to 9).

### Prior Art Dociments

### Non Patent Documents

[Non Patent Document 1] Trkola, A., Purtscher, M., Muster, T., Ballaun, C., Buchacher, A., Sullivan, N., Srinivasan, K., Sodroski, J., Moore, J. P., and Katinger, H., (1996), J Virol 70: 1100-1108.
[Non Patent Document 2] Haynes, B. F., Fleming, J., St Clair, E. W., Katinger, H., Stiegler, G., Kunert, R., Robinson, J., Scearce, R. M., Plonk, K., Staats, H. F., et al., (2005), Science 308: 1906-1908. Epub 2005 Apr 1928.
[Non Patent Document 3] Henriksson, G., Manthorpe, R., and Bredberg, A., (2000), Rheumatology (Oxford) 39: 142-147.
[Non Patent Document 4] Lenert, P., Lenert, G., and Senecal, J. L., (1996), Hum Immunol 49: 38-48.
[Non Patent Document 5] Lopalco, L., Magnani, Z., Confetti, C., Brianza, M., Saracco, A., Ferraris, G., Lillo, F., Vegni, C., Lazzarin, A., Siccardi, A. G, et al., (1999), AIDS Res Hum Retroviruses 15: 1079-1085.
[Non Patent Document 6] Rieber, E. P., Federle, C., Reiter, C., Krauss, S., Gurtler, L., Eberle, J., Deinhardt, F., and Riethmuller, G., (1992), Proc Natl Acad Sci U.S.A 89: 10792-10796.
[Non Patent Document 7] Benkirane, M., Hirn, M., Carriere, D., and Devaux, C., (1995), J Virol 69: 6898-6903.
[Non Patent Document 8] Moir, S., Lapointe, R., Malaspina, A., Ostrowski, M., Cole, C. E., Chun, T. W., Adelsberger, J., Baseler, M., Hwu, P., and Fauci, A. S., (1999), J Virol 73: 7972-7980.
[Non Patent Document 9] Kuritzkes, D. R., Jacobson, J., Powderly, W. G., Godofsky, E., DeJesus, E., Haas, F., Reimann, K. A., Larson, J. L., Yarbough, P. 0., Curt, V., et al., (2004), J Infect Dis 189: 286-291. Epub 2004 Jan 2008.

### Summary of Invention

### Problem to be Solved by the Invention

The mouse anti-CD4 monoclonal antibody described in Non Patent Documents 6 to 9 is likely to show broad cross-reactivity with multiple clades of HIV-1. It is considered that a human anti-CD4 antibody may play a certain role in protecting humans from HIV-1 infection or progression of AIDS. However, a human anti-CD4 monoclonal antibody, which is able to protect humans from infection with HIV such as HIV-1 or progression of AIDS caused by such HIV infection, has not yet been cloned. An agent capable of suppressing the replication of HIV, which comprises such an antibody as an active ingredient, and a pharmaceutical agent for preventing and/or treating AIDS, have not been known, either.

Hence, it is an object of the present invention to provide an agent for suppressing the replication of HIV, which comprises a human CD4-recognizing antibody. It is another object of the present invention to provide a pharmaceutical agent for preventing and/or treating AIDS, which comprises the above-mentioned agent for suppressing the replication of HIV as an active ingredient.

### Mans for Solving the Problem

In order to achieve the aforementioned objects, the present inventors have attempted to generate a human anti-CD4 monoclonal antibody from peripheral blood monocytes collected from healthy adult donors. As a result, the inventors have succeeded in isolating an independent clone of IgM Fab that recognizes human CD4. Subsequently, the present inventors have analyzed the sequence of the variable regions of the isolated antibody, and as a result, they have clarified that a variable region genes are selectively used to generate a human CD4-reactive antibody. That is to say, the antibody isolated by the present inventors was found to be a human autoreactive CD4 monoclonal antibody derived from a healthy person.

Furthermore, the present inventors have analyzed the influence of the above-described antibody upon the replication of HIV-1. As a result, they have revealed that the above-described antibody is able to suppress the replication of HIV-1 without impairing the original functions of human CD4. The present invention has been completed based on these findings.

Thus, the present invention provides an agent for suppressing the replication of human immunodeficiency virus, which comprises, as an active ingredient, an antibody described in (1) below:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing.

Another aspect of the present invention provides an agent for suppressing the replication of human immunodeficiency virus, which comprises, as an active ingredient, a nucleic acid comprising nucleotide sequences encoding an antibody described in (1) below:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing.

In a preferred embodiment of an agent for suppressing the replication of human immunodeficiency virus according to the present invention, the antibody recognizes an epitope present in a site of human CD4 that binds to gp120, which is different from the epitope recognized by Leu3a and/or RPA-T4.

In a preferred embodiment of an agent for suppressing the replication of human immunodeficiency virus according to the present invention; the antibody is a Fab antibody.

Another aspect of the present invention provides an agent for suppressing the replication of human immunodeficiency virus, which comprises, as an active ingredient, a nucleic acid described in (2) below:
(2) a nucleic acid comprising the nucleotide sequence shown in SEQ ID NO: 1 in the sequence listing and the nucleotide sequence shown in SEQ ID NO: 3 in the sequence listing.

In a preferred embodiment of an agent for suppressing the replication of human immunodeficiency virus according to the present invention, the nucleic acid comprised as an active ingredient is inserted into a vector.

In a preferred embodiment of an agent for suppressing the replication of human immunodeficiency virus according to the present invention, the vector is a plasmid vector or a virus vector.

In a preferred embodiment of an agent for suppressing the replication of human immunodeficiency virus according to the present invention, the human immunodeficiency virus is human immunodeficiency virus type 1.

Another aspect of the present invention provides a pharmaceutical agent for preventing and/or treating acquired immune deficiency syndrome, which comprises, as an active ingredient, the agent for suppressing the replication of human immunodeficiency virus according to the present invention.

Another aspect of the present invention provides an use of an antibody described in (1) below, a nucleic acid comprising nucleotide sequences encoding an antibody described in (1) below, or a nucleic acid described in (2) below, for the production of the agent for suppressing the replication of human immunodeficiency virus according to the present invention or the pharmaceutical agent according to the present invention:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing;
(2) a nucleic acid comprising the nucleotide sequence shown in SEQ ID NO: 1 in the sequence listing and the nucleotide sequence shown in SEQ ID NO: 3 in the sequence listing.

Another aspect of the present invention provides a method for suppressing the replication of human immunodeficiency virus which comprises administering, to mammal, an antibody described in (1) below, a nucleic acid comprising nucleotide sequences encoding an antibody described in (1) below, a nucleic acid described in (2) below, or the agent for suppressing the replication of human immunodeficiency virus according to the present invention:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing;
(2) a nucleic acid comprising the nucleotide sequence shown in SEQ ID NO: 1 in the sequence listing and the nucleotide sequence shown in SEQ ID NO: 3 in the sequence listing.

Another aspect of the present invention provides a method for preventing and/or treating acquired immune deficiency syndrome, which comprises administering, to mammal, the pharmaceutical agent according to the present invention.

### Advantageous Effects of Invention

The agent for suppressing the replication of HIV of the present invention comprises an antibody or a nucleic acid as an active ingredient. Such an antibody comprised as an active ingredient or an antibody encoded by such a nucleic acid comprised as an active ingredient recognizes human CD4 and binds thereto, and thus, it prevents the human CD4 from binding to HIV such as HIV-1, so that the present agent for suppressing the replication of HIV can suppress the replication of the HIV. Moreover, since it is highly likely that the above-described antibody recognizes a site in the human CD4, which does not impair the original functions of the human CD4, and binds thereto. Accordingly, it can be anticipated that the present antibody can suppress the replication of HIV, while preventing the occurrence of immune impairment caused by inhibition of human CD4 functions. Therefore, the pharmaceutical agent of the present invention, which comprises, as an active ingredient, the above-described agent for suppressing the replication of HIV, can prevent and/or treat AIDS in clinical sites.

### Brief Description of Drawings

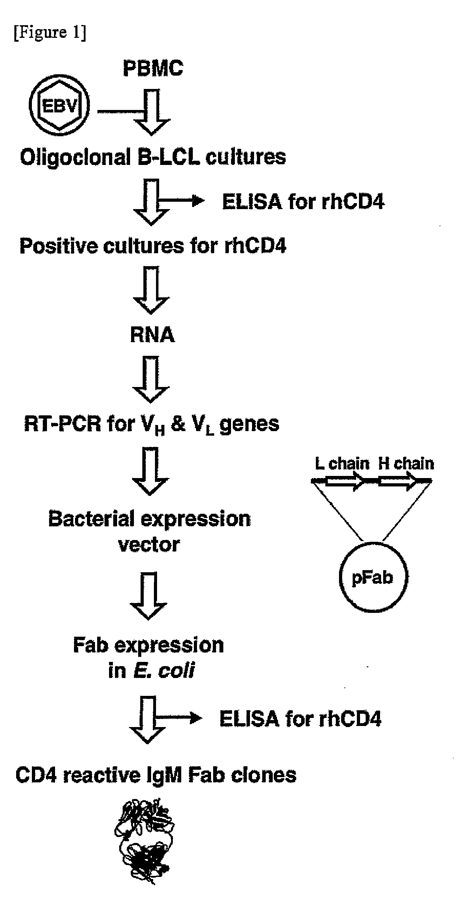

[Figure 1] Figure 1 is an experimental flow chart showing an outline of an experimental method for cloning human monoclonal CD4-reactive Fab genes.

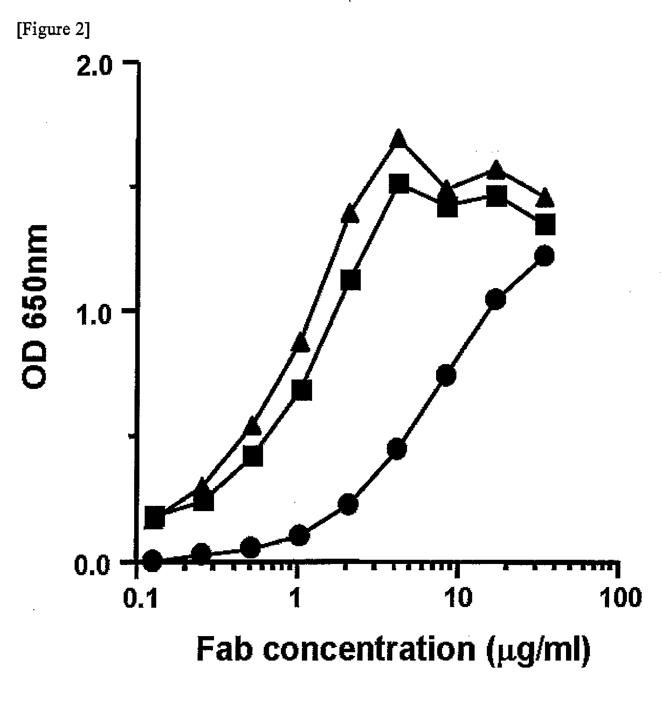

[Figure 2] Figure 2 is a view showing the results obtained by quantifying a human anti-CD4 antibody by ELISA. The figure shows the results obtained by incubating serially diluted HO538-213 (•), HO702-001 (■) and HO702-016 (▲) in a microtiter plate that had been pre-coated with rhCD4 (50 ng/well), and then quantifying the reaction products.

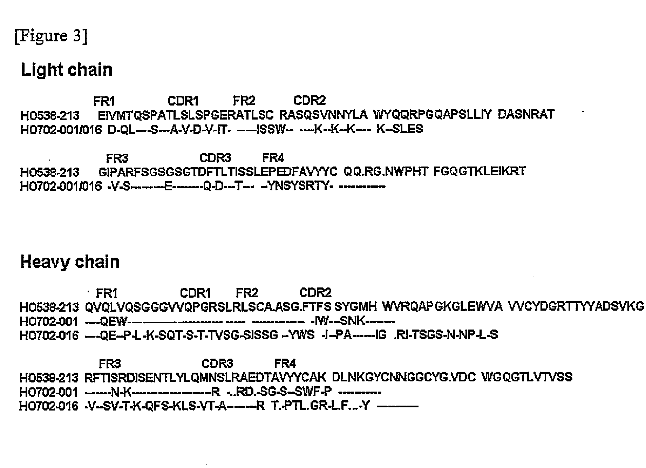

[Figure 3] Figure 3 is a view showing the alignment of the putative amino acid sequences of the V_{H} and V_{L} genes of each of human CD4-reactive Fab fragments HO538-213, HO702-001 and HO702-016. With regard to abbreviations used in the figure, "CDR" indicates a complementarity determining region, "FR" indicates a framework region, the symbol "-" indicates the same residue, and the symbol "•" indicates deletion of a residue.

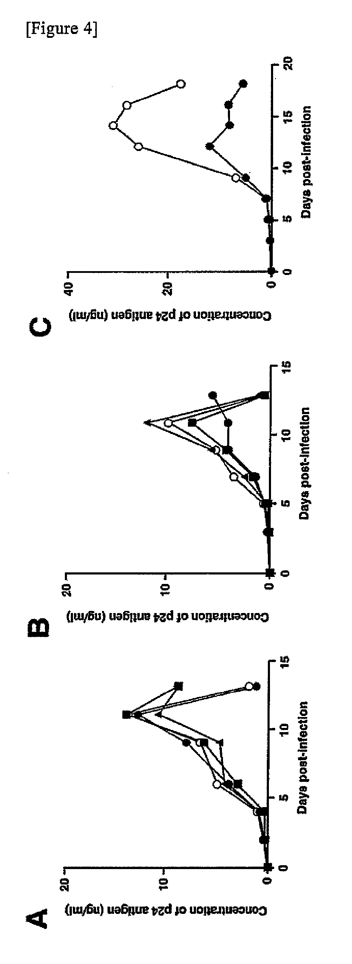

[Figure 4] Figure 4 shows the assay results obtained by examining the influence of human CD4-reactive Fab upon the replication of HIV-1_{JR-FL}. Fab clones HO538-213 (e), HO702-001 (■) and HO702-016 (▲) were examined in concentrations of 0.1 µ g/ml (A), 1.0µg/ml (B) and 2.5µg/ml (C). A human CD4-non-reactive Fab clone 13-3 (○) was used as a negative control.

### Embodiments for Carrying out the Invention

Hereinafter, the present invention will be described in detail.

The agent for suppressing the replication of human immunodeficiency virus (HIV) of the present invention comprises, as an active ingredient, an antibody recognizing human CD4 or a nucleic acid comprising nucleotide sequences encoding the aforementioned antibody, and thereby, the agent of the present invention can suppress the replication of the HIV. HIV belongs to family Retroviridae, genus Lentivirus, which is a plus strand, single-stranded RNA virus having an envelope. To date, HIV-1 (human immunodeficiency virus type 1) and HIV-2 (human immunodeficiency virus type 2) have been known as such HIV. The basic gene structure of HIV is almost the same as that of HIV-2. However, their nucleotide sequences have low homology (homology of approximately 60%). HIV-1 has infectivity higher than that of HIV-2, and it has a short incubation period until the onset of acquired immune deficiency syndrome (AIDS). Thus, HIV-1 has been spread over the world. Such HIV-1 is a preferred target of the agent for suppressing the replication of HIV of the present invention. Hereinafter, the present invention will be described step by step.

### [1] Human CD4

Human CD4 is a single chain transmembrane glycoprotein having a molecular weight of approximately 59 kDa. Human CD4 binds to a non-polymorphic region of an MHC class II molecule, and it may become a co-receptor in MHC class II-restricted antigenic stimulation and activation. It has been known that human CD4 is expressed in specific subsets of peripheral blood lymphocytes, namely, helper T cells or monocytes. A human CD4-positive T cell subset exhibits activity of inducing and/or promoting the synthesis of immunoglobulins by B cells. Human CD4 functions as a receptor (acceptor) of gp120 that is an envelope glycoprotein of human immunodeficiency virus type 1 (HIV-1). Accordingly, whether or not a certain protein is human CD4 can be confirmed by ELISA using gp120.

The type of human CD4, which can be used to obtain an antibody recognizing the human CD4 or to confirm that the obtained antibody recognizes the human CD4, is not particularly limited. Moreover, a method of obtaining such human CD4 is not particularly limited, either. For instance, there can be used human CD4, which is produced by a generally known genetic engineering method or physicochemical method based on the genetic information or protein information of human CD4-positive T cells. Specifically, Baculovirus-derived recombinant human CD4 (rhCD4) can be procured from American Research Products, Inc. (catalog No. 18-13001).

### [2] Antibody that recognizes human CD4

The antibody that recognizes human CD4 is an antibody that specifically recognizes human CD4. It is specifically an antibody described in (1) below. (1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing.

As described below, SEQ ID NOS: 2 and 4 in the sequence listing show the amino acid sequence of the variable region of the heavy chain of an antibody recognizing human CD4 and the amino acid sequence of the variable region of the light chain of an antibody recognizing human CD4, respectively.
SEQ ID NO: 2: the amino acid sequence of the variable region of the heavy chain of an antibody recognizing human CD4
SEQ ID NO: 4: the amino acid sequence of the variable region of the light chain of an antibody recognizing human CD4

In general, an antibody consists of two types (large and small) of polypeptides. The subunit of the larger polypeptide is called "H chain" (heavy chain), whereas the subunit of the smaller polypeptide is called "L chain" (light chain). Each peptide is composed of a "variable region" that is present on the N-terminal side and forms an antigen binding site, and a certain "constant region" that depends on the class of the antibody. The variable region is further divided into complementarity determining regions "CDR" that are closely associated with, in particular, the formation of the antigen binding site, and "frameworks" existing among them. It has been known that such CDR includes three regions called "CDR1," "CDR2" and "CDR3" from the N-terminal side in each of the H chain and the L chain.

The antibody that recognizes human CD4 includes: Fab consisting of a pair of a H chain fragment and a L chain fragment; F(ab')₂ consisting of two pairs of a H chain fragment and a L chain fragment; a single-stranded antibody in which a H chain fragment is tandemly bound to a L chain fragment in a single peptide; as well as an antibody that is generally present *in vivo.* The antibody that recognizes human CD4 may also be a full-length antibody generally existing *in vivo,* which is composed of two pairs of a full-length H chain and a full-length L chain.

With regard to the antibody described in (1) above, the range of "one or multiple" in the description "at least one type of mutation selected from the group consisting of deletion, substitution, inversion, addition and insertion of one or multiple amino acids" is not particularly limited, as long as it is the range in which the antibody described in (1) above can specifically recognize human CD4. The range of such "one or multiple" is, for example, 1 to 20, preferably 1 to 10, more preferably 1 to 7, further preferably 1 to 5, and particularly preferably 1 to 3. Moreover, "deletion of amino acids" means the lack or disappearance of amino acid residue in the sequence, "substitution of amino acids" means that amino acid residue in the sequence is substituted with another amino acid residue, and "inversion of amino acids" means that the positions of two or more amino acid residues adjacent to one another are inverted. Furthermore, "addition of amino acids" means that amino acid residue is added to the sequence, and "insertion of amino acid" means that another amino acid residue is inserted between amino acid residues in the sequence, respectively.

The method of obtaining the antibody of (1) above is not particularly limited. For example, the antibody of (1) above may be physicochemically synthesized with reference to the amino acid sequences shown in SEQ ID NOS: 2 and 4 in the sequence listing, or it may be obtained from cells that express the antibody of (1) above according to a method of inducing mutagenesis or the like. Alternatively, the antibody of (1) above may be produced from a nucleic acid comprising nucleotide sequences encoding the antibody of (1) above according to genetic engineering procedures based on the information of SEQ ID NOS: 1 and 3 in the sequence listing. Otherwise, the antibody of (1) above may also be obtained by various types of screening methods using other known means.

The antibody of (1) above is more preferably an antibody that recognizes an epitope present in a site of human CD4 that binds to gp120, which is different from the epitope recognized by Leu3a and/or RPA-T4. When this antibody binds to human CD4, it can be anticipated to inhibit the binding of the human CD4 to HIV without impairing the original functions of the human CD4. Human CD4 neutralizing antibodies such as Leu3a and RPA-T4 inhibit the binding of human CD4 to other ligands necessary for physiological functions. Thus, such human CD4 neutralizing antibodies are likely to inhibit the physiological functions of human CD4. In other words, if a certain antibody is not antagonistic against the human CD4 neutralizing antibody, it means that the certain antibody does not have the ability of the human CD4 neutralizing antibody. Thus, even if the certain antibody binds to human CD4, it is considered that it does not inhibit the physiological functions of the human CD4.

In a preferred embodiment, the antibody of (1) above is a Fab antibody. The Fab antibody of (1) above can be obtained by screening a bacterial Fab expression library, which is produced based on the information of RNA obtained from healthy adult-derived peripheral blood monocyte-derived cells that produce human anti-CD4 antibodies, for example, from B-lymphoblastic cell line (which is also referred to as "B-LCL"). Moreover, the fact that the human CD4-reactive Fab expression clone obtained by the screening can suppress the replication of HIV can be confirmed by adding the human CD4-reactive Fab expression clone to a culture supernatant of suitable cells, such as 293T cells transformed with HIV provirus DNA, then incubating the mixture, and then detecting an HIV marker protein such as p24 in the culture supernatant obtained after completion of the incubation. Hereafter, a specific screening method and a confirmation method will be described with reference to the descriptions of examples.

First, peripheral blood monocytes are collected from healthy adults, and the collected monocytes is then infected with EBV (Epstein-Barr virus) and then B-lymphoblastic cell lines (B-LCL) are established and are cultured in a cancerous state. Using a supernatant obtained by this culture, a cell line that produces an antibody recognizing human CD4 is selected. This selection can be carried out using human CD4 as an antigen, by a method well known to persons skilled in the art, such as an immunostaining method, an enzyme-linked immunoassay (ELISA), a double monoclonal antibody sandwich immunoassay (U.S. Patent No. 4,376,110), a monoclonal polyclonal antibody sandwich assay (Wide et al., edited by Kirkham and Hunter, "Radioimmunoassay", E. and S. Livingstone, Edinburgh (1970)), or an immunoprecipitation method. The selection is preferably carried out by ELISA because this method has high sensitivity and is commonly used.

ELISA includes a direct adsorption method, a sandwich method, and a competition method. The direct adsorption method will be described below as a specific example. First, a solution containing recombinant human CD4 (rhCD4) as an antigen is added to wells of an ELISA plate. After the rhCD4 has been removed, the washing of the wells, blocking and the like are carried out as appropriate. Subsequently, a test sample that is likely to contain an anti-rhCD4 antibody directed against rhCD4 is added to the wells. After the test sample has been removed, a labeled secondary antibody that recognizes the anti-rhCD4 antibody is added to the wells. After the labeled secondary antibody has been removed, a complex of the rhCD4, the anti-rhCD4 antibody and the secondary antibody is detected using the labeling.

Thereafter, oligoclonal cells that are positive in production of a human anti-CD4 antibody are selected, and total RNA is then isolated from these cells. A method of isolating total RNA from the cells is not particularly limited. For example, RNeasy Mini Kit (QIAGEN) can be used. The isolated RNA is subjected to a reverse transcription polymerase chain reaction (RT-PCR), using primers specific to the genes of various human immunoglobulin chains, such as Igµ, γ, λ and κ (SEQ ID NOS: 5 to 14 in the sequence listing), so as to synthesize and amplify cDNA.
SEQ ID NO: 5 in the sequence listing; VH1a; 5' primer for H chain (for both γ chain and µ chain)
SEQ ID NO: 6 in the sequence listing; VH1b; 5' primer for H chain (for both γ chain and µ chain)
SEQ ID NO: 7 in the sequence listing; VH2a; 5' primer for H chain (for both γ chain and µ chain)
SEQ ID NO: 8 in the sequence listing; VH3; 5' primer for H chain (for both γ chain and µ chain)
SEQ ID NO: 9 in the sequence listing; VH4c; 5' primer for H chain (for both γ chain and µ chain)
SEQ ID NO: 10 in the sequence listing; FDG1; 3' primer for γ chain
SEQ ID NO: 11 in the sequence listing; FDG2; 3' primer for γ chain
SEQ ID NO: 12 in the sequence listing; FDG3; 3' primer for γ chain
SEQ ID NO: 13 in the sequence listing; FDG4; 3' primer for γ chain
SEQ ID NO: 14 in the sequence listing; FDM; 3' primer for µ chain
SEQ ID NO: 15 in the sequence listing; VK1; 5' primer for κ chain
SEQ ID NO: 16 in the sequence listing; VK2a; 5' primer for κ chain
SEQ ID NO: 17 in the sequence listing; VK3a; 5' primer for κ chain
SEQ ID NO: 18 in the sequence listing; VK4; 5' primer for κ chain
SEQ ID NO: 19 in the sequence listing; VKC; 3' primer for κ chain
SEQ ID NO: 20 in the sequence listing; VL1a; 5' primer for λ chain
SEQ ID NO: 21 in the sequence listing; VL1b; 5' primer for λ chain
SEQ ID NO: 22 in the sequence listing; VL2a; 5' primer for λ chain
SEQ ID NO: 23 in the sequence listing; VL2b; 5' primer for λ chain
SEQ ID NO: 24 in the sequence listing; VL3a; 5' primer for λ chain
SEQ ID NO: 25 in the sequence listing; VL3b; 5' primer for λ chain
SEQ ID NO: 26 in the sequence listing; VLC; 3' primer for λ chain

It is preferable to use touch-down PCR as this PCR. In the touch-down PCR, there is used a gene-specific primer (GSP) having a melting temperature (Tm) slightly higher than the melting temperature (Tm) of a universal primer, and the annealing temperature is set at the Tm of GSP during several initial cycles of the PCR. As a result, only synthesis specific to a gene primed by GSP occurs, and thus, only a gene of interest is concentrated from the template cDNA at the initial stage of the PCR. In the subsequent cycles, the annealing temperature is decreased to the Tm of the universal primer, and a specific amplification of the gene of interest is then carried out. According to the touch-down PCR, a small amount of transcription product (e.g. a transcription product from approximately 50 ng of total RNA) can be amplified from either poly(A) RNA or total RNA.

Thereafter, the amplified product is cloned into a suitable vector to produce a Fab expression library. As such a vector, there is generally used a vector suitable for a host, in which the vector is to be expressed. When the vector is to be expressed in *Escherichia coli,* for example, a pFab1-His2 vector and the like can be used.

Subsequently, a host is transformed with the produced Fab expression library, and a human CD4-reactive Fab expression clone is then screened from the obtained transformants. As described above, ELISA or the like can be applied to this screening operation. Examples of a method of purifying a Fab fragment from the clone include: methods utilizing a difference in molecular weight, such as dialysis, ultrafiltration, resin column, gel filtration, gel filtration chromatography and SDS-polyacrylamide gel electrophoresis; methods utilizing a difference in solubility, such as salt precipitation, ammonium sulfate precipitation, alcohol precipitation and other solvent precipitations; methods utilizing a difference in electric charge, such as ion exchange chromatography using DEAE-Toyopearl resin and the like; methods utilizing specific affinity, such as affinity chromatography; methods utilizing a difference in hydrophobicity, such as hydrophobic chromatography using butyl-Toyopearl resin and the like and reverse phase chromatography; methods utilizing a difference in the degree of physicochemical adsorption, such as adsorption chromatography; and methods utilizing a difference in isoelectric point, such as isoelectric focusing electrophoresis and isoelectric chromatography. These commonly used methods can be used singly or in combination.

Whether or not the antibody recognizing human CD4 can suppress the replication of HIV can be confirmed according to the method described in examples, for example. In the method described in examples, primary cultured monocytes are first incubated at 37°C for 30 minutes, together with a culture supernatant of 293T cells that generates infectious virions of JR-FL strains as a result of introduction of HIV-1 provirus DNA. Thirty minutes later, approximately 0.9 to 2 ng of HIV-1 is preferably contained in the culture supernatant, using p24 as a marker. Subsequently, the 24p antigen is detected in the culture supernatant according to a generally known method such as ELISA. When the detection is carried out by ELISA, the operations can be carried out using RETRO-TEK HIV-1 p24 Antigen ELISA Kit (ZeptoMetrix) in accordance with the protocols provided by the manufacturer.

### [3] Nucleic acid comprising nucleotide sequence encoding antibody recognizing human CD4

A nucleic acid comprising nucleotide sequences encoding an antibody recognizing human CD4 comprises nucleotide sequences encoding the antibody described in (1) above. More specifically, it is a nucleic acid comprising the nucleotide sequence shown in SEQ ID NO: 1 in the sequence listing and the nucleotide sequence shown in SEQ ID NO: 3 in the sequence listing. As described below, SEQ ID NOS: 1 and 3 in the sequence listing show nucleotide sequences corresponding to the amino acid sequence of the variable region of the heavy chain of an antibody recognizing human CD4 and the amino acid sequence of the variable region of the light chain of an antibody recognizing human CD4, respectively.
SEQ ID NO: 1: a nucleotide sequence corresponding to the amino acid sequence of the variable region of the heavy chain of an antibody recognizing human CD4
SEQ ID NO: 3: a nucleotide sequence corresponding to the amino acid sequence of the variable region of the light chain of an antibody recognizing human CD4

The method of obtaining a nucleic acid comprising nucleotide sequences encoding an antibody recognizing human CD4 is not particularly limited. For example, the nucleic acid may be physicochemically synthesized with reference to the amino acid sequences shown in SEQ ID NOS: 2 and 4 in the sequence listing, or it may be obtained from cells that express the antibody of (1) above according to a method of inducing mutagenesis or the like. Alternatively, it may be produced according to genetic engineering procedures based on the information of SEQ ID NOS: 1 and 3 in the sequence listing. Otherwise, it may also be obtained by various types of screening methods using other known means.

An example of the a nucleic acid comprising the nucleotide sequences encoding the antibody recognizing human CD4 may be a nucleic acid that hybridizes under stringent conditions with a nucleic acid comprising the nucleotide sequence shown in SEQ ID NO: 1 in the sequence listing and the nucleotide sequence shown in SEQ ID NO: 3 in the sequence listing. The nucleic acid that "hybridizes under stringent conditions" means a nucleic acid obtained by applying a colony hybridization method, a plaque hybridization method, a Southern blot hybridization method or the like, using DNA as a probe. Such a nucleic acid is, for example, DNA that can be identified by performing hybridization at 65°C in the presence of 0.7 to 1.0 M NaCl, using a filter, on which colony- or plaque-derived DNA or a DNA fragment thereof has been immobilized, and then washing the filter at 65°C with a 0.1 to 2 × SSC solution (wherein a 1 × SSC solution consists of 150 mM sodium chloride and 15 mM sodium citrate). Genetic engineering methods and molecular biological methods applied in the present specification, including the above-mentioned hybridization, can be carried out in accordance with generally known methods described in Molecular Cloning: A laboratory Manual, 2nd Ed., Cold Spring Harbor Laboratory, Cold Spring Harbor, NY, 1989, Current Protocols in Molecular Biology, Supplement 1 to 38, John Wiley & Sons (1987-1997), etc.

In a preferred embodiment, the nucleic acid comprising the nucleotide sequences encoding the antibody recognizing human CD4 is recombinant DNA inserted into an autonomously replicating vector. The type of such a vector is not particularly limited, as long as it is a vector that is able to autonomously replicate in human cells. Examples of such a vector include expression vectors such as pGL4.12 and virus vectors. Virus vectors are preferable. Examples of such a virus vector include an adenovirus vector, a lentivirus vector, a herpes simplex virus vector, and a retrovirus vector. As a method of inserting the nucleic acid comprising the nucleotide sequence encoding the antibody recognizing human CD4 into a vector, a generally known method described in the aforementioned Molecular Cloning: A laboratory Manual, 2nd Ed., Cold Spring Harbor Laboratory, Cold Spring Harbor, NY., 1989, Current Protocols in Molecular Biology, Supplement 1 to 38, John Wiley & Sons (1987-1997), etc. can be applied without any restrictions.

### [4] Agent for suppressing replication of HIV

The agent for suppressing the replication of HIV of the present invention comprises, as an active ingredient, the above-described antibody recognizing human CD4 or the above-described nucleic acid comprising nucleotide sequences encoding the antibody recognizing human CD4, so that it can suppress the replication of HIV.

When the agent for suppressing the replication of HIV of the present invention is allowed to come into contact with a site in which HIV is present, such as the body fluid or cells of a mammal (hereinafter also referred to a location of HIV), if the above-described antibody as an active ingredient or an antibody encoded by the above-described nucleic acid as an active ingredient can suppress the replication of the HIV in the location of the HIV, the mass ratio (the antibody or nucleic acid/the replication-suppressing agent) of the antibody recognizing human CD4 or the nucleic acid comprising nucleotide sequences encoding the antibody recognizing human CD4, which is contained in the expression-suppressing agent of the present invention, is not particularly limited. For example, the mass ratio is preferably 0.5 to 0.99, more preferably 0.8 to 0.99, and further preferably 0.9 to 0.99. The agent for suppressing the replication of HIV of the present invention may substantially comprise 100% of the above-described antibody or nucleic acid.

The agent for suppressing the replication of HIV of the present invention can be used in the form of either a solid or a liquid, as long as it comprises an effective amount of antibody recognizing human CD4 or nucleic acid comprising nucleotide sequences encoding the antibody recognizing human CD4. Alternatively, a pharmaceutically acceptable carrier or additive may be mixed with the agent for suppressing the replication of HIV of the present invention, so as to prepare a pharmaceutical agent that is in the form of a solid or a liquid. The agent for suppressing the replication of HIV of the present invention can be produced by mixing the above-described antibody or nucleic acid with another ingredient by a generally known method.

In the use of the agent for suppressing the replication of HIV of the present invention, the method of allowing the agent for suppressing the replication of HIV of the present invention to come into contact with the location of HIV is not particularly limited, as long as the antibody recognizing human CD4 or the nucleic acid comprising nucleotide sequences encoding the antibody recognizing human CD4, which is comprised as an active ingredient, is allowed to come into contact with HIV in the location of HIV. For example, a known method of directly introducing the agent for suppressing the replication of HIV of the present invention into the location of HIV, such as electroporation, or a method of adding the agent for suppressing the replication of HIV of the present invention to the location of HIV in the form of being easily incorporated into the location of HIV, such as a liposome, can be applied.

### [5] Pharmaceutical agent for preventing and/or treating AIDS

The present invention includes a pharmaceutical agent for preventing and/or treating AIDS, which comprises, as an active ingredient, the agent for suppressing the replication of HIV of the present invention.

The target disease, to which the pharmaceutical agent of the present invention can be applied, is AIDS. Such AIDS is interpreted to be generally known AIDS. Thus, the term AIDS can be used to mean immunodeficiency disease, which is caused by infecting human CD4-positive T cells with HIV and then destroying the human CD4-positive T cells by the HIV. The pharmaceutical agent of the present invention impedes the binding between human CD4 and HIV, and thereby prevents the infection of human CD4-positive T cells with the HIV, so as to prevent and/or treat AIDS.

For example, among patients with AIDS, those having moderate symptoms can be prevented from the progression or deterioration of the symptoms by administration of the pharmaceutical agent of the present invention. Even in the case of AIDS patients having severe symptoms, there may also be a case in which the therapeutic effects of the present pharmaceutical agent can be anticipated. Moreover, since the HIV virus that causes AIDS is a mother-to-child transmission type virus, a child born from an HIV-infected mother can be prevented from the onset of AIDS by being administered with the pharmaceutical agent of the present invention.

As the pharmaceutical agent of the present invention, the agent for suppressing the replication of HIV of the present invention may be directly used. In general, however, it is desired to prepare a pharmaceutical composition comprising the agent for suppressing the replication of HIV of the present invention as an active ingredient and one or two or more additives for agent formulation, and to use it.

For prevention and/or treatment of AIDS, it is also desired to use a cytokine or the like for enhancing immunity in combination with the pharmaceutical agent of the present invention, or to implant undifferentiated lymphocytes or the like.

Examples of the form of a pharmaceutical composition suitable for oral administration include a tablet, a capsule, a powdered drug, a fine grain agent, a granule, a liquid agent, and a syrup. Examples of the form of a pharmaceutical composition suitable for parenteral administration include an injection, a drop, a suppository, an inhalant, an eye drop, a nasal drop, an ointment, a cream, a patch, a transdermal agent, and a transmucosal agent. Examples of an additive for agent formulation, which is used in the production of the above-described pharmaceutical composition, include an excipient such as lactose or oligosaccharide, a disintegrator or a disintegration aid, a binder, a lubricant, a coating agent, a pigment, an antioxidant, a flavoring agent, a diluent, a base, a dissolving agent or a solubilizing agent, an isotonizing agent, a preservative, a pH adjuster, a stabilizer, an isotonizing agent, a propellant, an emulsifier, a suspending agent, a solvent, a filler, a thickener, a buffer, a delivery vehicle, a carrier, a pharmaceutical adjuvant, and an adhesive. These additives may be selected, as appropriate, by persons skilled in the art, depending on the form of the pharmaceutical composition. Also, two or more types of additives may be used in combination.

A preferred form of the pharmaceutical agent of the present invention is an injection. Such an injection can be dissolved in or diluted with a solvent, which, in general, substantially does not comprise a non-aqueous solvent (or a water-soluble organic solvent) and in which the medium is substantially water. Such an injection can be prepared according to a method well known in the present technical field. For example, such an injection can be prepared by dissolving the pharmaceutical agent of the present invention in a solvent such as a normal saline, a buffer such as PBS, or a sterile water, then sterilizing the obtained solution by filtration using a filter or the like, and then filling the resultant solution into an aseptic vessel (e.g. an ampule, etc.). The thus prepared injection may comprise a commonly used pharmaceutical carrier, as necessary. In addition, an administration method using a non-invasive catheter may be applied. Examples of the carrier that can be used in the present invention include a neutral buffered normal saline and a normal saline containing serum albumin.

Another preferred form of the pharmaceutical agent of the present invention is a freeze-dried agent (a freeze-dried injection). Even such a freeze-dried agent can be dissolved in at least one liquid or solvent selected from among water used for injection (distilled water used for injection), an infusion solution comprising an electrolyte liquid (a normal saline, etc.), an infusion of nutrient, and the like, and thus, an injection can be easily prepared from the freeze-dried agent. As a vessel, both a glass vessel and a plastic vessel can be used. The expression-suppressing agent of the present invention can be added in an amount of 0.01 part by weight or more, and preferably 0.1 to 10 parts by weight, with respect to 100 parts by weight of the content of the injection.

The applied dose and number of doses of the pharmaceutical agent of the present invention are not particularly limited. Such applied dose and number of doses can be selected, as appropriate, depending on conditions such as the age, body weight and sex of a patient, and also depending on the type of disease, severity, the purpose of prevention or treatment, etc. In general, in the case of parenteral administration, the applied dose of the active ingredient of the pharmaceutical agent of the present invention is preferably 0.5 µg/ml to 10 mg/ml, more preferably 1 µg/ml to 1 mg/ml, and further preferably 10 µg/ml to 0.5 mg/ml, per adult per day. The applied dose may be divided into several administrations per day. The administration frequency of the pharmaceutical agent of the present invention may be, for example, once per day to once per several months, and preferably once per month.

The agent for suppressing the replication of HIV or pharmaceutical agent of the present invention cannot be used only in the form of a pharmaceutical product as described above, but it can also be used as a quasi drug, a cosmetic product, a functional food, a nutritional supplementary food, food and drink, etc. When the agent for suppressing the replication of HIV or pharmaceutical agent of the present invention is used as a quasi drug or a cosmetic product, it may be used, as necessary, together with various auxiliary agents that are commonly used in the technical field of quasi drugs, cosmetic products, etc. On the other hand, when it is used as a functional food, a nutritional supplementary food, or food and drink, it may be used, as necessary, together with additives commonly used in food products, such as a sweetener, a spice, a condiment, an antiseptic, a preservative, a disinfectant or an antioxidant. The agent for suppressing the replication of HIV or pharmaceutical agent of the present invention may also be used in a desired form such as a solution, a suspension, a syrup, a granule, a cream, a paste or a jelly. Otherwise, it may also be molded and then used, as necessary. The ratio of these additives contained is not particularly limited, and it can be selected, as appropriate, depending on intended use, the type of usage, and an amount used.

Hereinafter, the present invention will be described more in detail in the following examples. However, these examples are not intended to limit the scope of the present invention.

### Examples

### 1. Materials and methods

### (1) Functional cloning of antibody heave-chain and light-chain genes

The establishment of antibody-producing cells, the cloning of an immunoglobulin gene encoding a variable region, ELISA, and purification of a Fab fragment from *Escherichia coli* were carried out in accordance with the previous reports (Takekoshi, M., Maeda, F., Tachibana, H., Inoko, H., Kato, S., Takakura, I., Kenjyo, T., Hiraga, S., Ogawa, Y., Horiki, T., et al., (1998), J Virol Methods 74: 89-98.; Takekoshi, M., Maeda, F., Nagatsuka, Y., Aotsuka, S., Ono, Y., and Ihara, S., (2001), J Biochem 130: 299-303.). The summary thereof will be described below. Peripheral blood monocytes were infected with EBV (Epstein-Barr Virus), strain B95-8, and the cells were then allowed to grow in a multi-well plate. Thereafter, the obtained supernatant was analyzed by ELISA, using baculovirus-drived rhCD4 (50 ng/well, INTRACEL) as an antigen.

ELISA was carried out as follows.

The rhCD4 was dissolved in a 50 mM carbonate buffer (pH 9.6) solution to a concentration of 2 µg/mL, and the thus prepared solution was dispensed in an amount of 50 µL each into the wells on an ELISA plate, and it was then left at 4°C overnight. Thereafter, the wells were washed with PBS-T (PBS-0.05% Tween) once. Then, 300 µL of PBS-T-SM (PBS-T-1% skim milk) was added thereto, so as to block it at room temperature for 1 hour. Subsequently, the PBS-T-SM was removed, 50 µL of an antibody sample diluted with PBS-T-SM was then added thereto, and the mixture was then left at room temperature for 1 hour. Thereafter, the sample was removed, and the residue was then washed with PBS-T three times. A PO-bound anti-human IgG-Fab antibody was diluted with PBS-T at a ratio of 1 : 2000, and 50 µL of the thus diluted solution was then added to the wells. The obtained mixture was left at room temperature for 1 hour. Thereafter, the secondary antibody was removed, and the residue was then washed with PBS-T three times. 50 µL of a TMB reagent was added to the resultant, so as to develop color. The color was measured at OD of 650 nm.

Total RNA was isolated from an oligoclonal cell population that had tested positive for the production of a human anti-CD4 antibody (RNeasy Mini Kit, Qiagen). Using primers specific to human Igµ, γ, λ and κ chains (SEQ ID NOS: 5 to 26 in the sequence listing), cDNA was synthesized and amplified by a polymerase chain reaction (PCR). The specific primers were each adjusted to a final concentration of 10 pmol/µL. In a case in which multiple primers were present, they were mixed in equal amounts, so as to adjust them to the aforementioned final concentration. In accordance with the descriptions of the aforementioned publications, the protocols of "touch-down" PCR were applied (Invitrogen; One-step RT-PCR with Platinum Taq; catalog No. 10928-042). The PCR was carried out by the program shown in the following Table 1.

**Table 1**

| RT | | PCR | | | | | | | |
|---|---|---|---|---|---|---|---|---|---|
| 50→ | 94 | 95→ | 65,64,,,,,,56, | 72→ | 94→ | 55→ | 72→ | 72→ | 20 |
| | → | | 55→ | | | | | | |
| 20m | 2m | 1m | 1m* | 2m | 1m | 1m | 2m | 10m | ∞ |
| | | | 11 cycles* | | | 25 cycles* | | | |

| | | | | | | | | | |
|---|---|---|---|---|---|---|---|---|---|
| * The temperature was decreased by 1°C for each step from 65°C to 55°C (11 cycles). After the temperature was decreased to 55°C, then 25 cycles were carried out under the same conditions. | | | | | | | | | |

The amplified µ and γ chains and the amplified λ and κ chains were incorporated into the *Sfi* I/*Not* I sites and the *Nhe* I/*Asc* I sites of a pFabl-His2 vector respectively so as to prepare a bacterial Fab expression library (Maeda, F., Nagatsuka, Y., Ihara, S., Aotsuka, S., Ono, Y., Inoko, H., and Takekoshi, M., (1999), J Med Virol 58: 338-345.). An *Escherichia coli* JM109 strain was transformed with the pFab library, and human CD4-reactive Fab expression clones were screened by ELISA. The Fab fragment was purified using an anti-Fab antibody affinity column. The eluted Fab was dialyzed against PBS, and was then centrifuged (VIVASPIN centrifuge tube, Vivascience AG). The purity of the Fab fragment exceeded 95%, when analyzed by sodium dodecyl sulfate-polyacrylamide gel electrophoresis (SDS-PAGE).

### (2) Cells

B-LCL and 293T cells were maintained in RPMI 1640 (Sigma) to which 10% FBS (Japan Bio Serum), penicillin and streptomycin (Invitrogen) had been added. Primary cultured monocytes were maintained in RPMI 1640, to which 10% FBS, penicillin, streptomycin, 5 µg/ml Plasmocin (InvivoGen), a 5 µg/ml anti-CD3 monoclonal antibody (OKT3, Janssen Pharmaceutical K.K.), 70 U/ml recombinant human interleukin 2 (rhIL-2, Shionogi & Co., Ltd.), GlutaMax-I (Invitrogen), insulin-transfernn-selenium A (Invitrogen), and 10 mM HEPES (Invitrogen) had been added. The cells were cultured under humidification conditions of 37°C and 5% CO₂.

### (3) Monitoring of replication of HIV-1

The assay was carried out by the procedures of the previous reports (Shimizu, S., Urano, E., Futahashi, Y., Miyauchi, K., Isogai, M., Matsuda, Z., Nohtomi, K., Onogi, T., Takebe, Y., Yamamoto, N., et al., (2007), AIDS 21: 575-582.). The summary thereof will be described below. Primary cultured monocytes (1 × 10⁵ cells) were incubated at 37°C for 30 minutes, together with a culture supernatant (which contained HIV-1 [approximately 0.9 to 2 ng of p24]) of 293T cells, which generated infectious virions of JR-FL strains as a result of introduction of HIV-1 provirus DNA. Thereafter, the culture supernatant was measured by ELISA, and a p24 antigen was detected. For such ELISA, RETRO-TEK HIV-1 p24 Antigen ELISA Kit (ZeptoMetrix) was used in accordance with the protocols of the manufacturer.

### 2. Results

Peripheral blood monocytes were collected from one healthy adult volunteer. A B-lymphoblastic cell line (B-LCL) was established by infecting the cells with Epstein-Barr virus (EBV) (Figure 1). The B-LCL transformed with the EBV was allowed to grow to be oligoclonal. The reactivity of the culture supernatant with recombinant human CD4 (rhCD4) was analyzed by ELISA. Two cell populations (HO538 and HO702) tested positive for rhCD4 reactivity were identified. Since these cell populations did not react with other screened antigens, the reactivity thereof with rhCD4 was specific. The cells in the positive wells were allowed to grow, and RNA was then extracted from the cells. A gene encoding a Fab antibody fragment was amplified by RT-PCR, and it was then cloned into a bacterial expression vector pFabl-His2 that was to produce a Fab fragment of the inserted heavy and light chain gene set. It was anticipated that human CD4-reactive Fab present in the initial B-LCL cell population would be reconstituted in several clones. After completion of the screening operation by ELISA, human CD4-reactive IgM Fab clones were isolated. That is, one clone (HO538-213) was isolated from the HO538 cell population, and two independent clones (HO702-001 and HO702-016) were isolated from the HO702 cell population. Hereinafter, descriptions regarding HO538-213 are provided as an example, and descriptions regarding HO702-001 and HO702-016 are provided as comparative examples.

As a result of the analysis of nucleotide sequences, these clones were found to be IgM Fab. The percentage of peripheral B cells producing human CD4-reactive antibodies in total peripheral B cells was assumed to be approximately 0.0013%. The binding of the IgM Fab of each of the purified three clones to rhCD4 was examined by ELISA (Figure 2). Based on the dilution magnification at which the absorbance at OD of 650 nm became approximately 0.7 to 0.8 (which was a value at which 50% of the maximum binding was observed), the amount of the antibody was assumed to be approximately 1 µg/ml in the case of HO702-001 and HO702-016, and it was assumed to be approximately 8 µg/ml in the case of HO538-213. The affinity of HO538-213 for rhCD4 was 1/8 of other clones.

The Fab sequences were analyzed using the Kabat database (http://www.nobi.nlm.nih.gov/igblast/) of GenBank, as described in 1(1) above. With regard to each gene, the immunoglobulin (Ig) gene family and the closest germ cell gene are shown in Table 2 below.

**Table 2**

| clone | Ig class | VH family | closest germline | percentage identity | VL family | closest germline | percentage identity |
|---|---|---|---|---|---|---|---|
| HO538-213 | IgM | VH3 | VH3-33 | 95% | Vk3 | L6 | 97% |
| HO702-01 | IgM | VH3 | VH3-33 | 97% | Vk1 | L12 | 97% |
| HO702-16 | IgM | VH4 | VH4-4 | 96% | Vk1 | L12 | 97% |

As a result of the sequence analysis, light chain was found to be κ chain in all of the three clones. When the three clones were compared with the germ cell genes in terms of heavy chain, the µ chains of HO538-21 and HO702-001 showed homology of 95% and 97% with the germ cell gene V_{H} 3-33, respectively. The µ chain of HO702-016 showed homology of 96% with the germ cell gene V_{H} 4-4 (Matsuda, F., Ishii, K., Bourvagnet, P., Kuma, K., Hayashida, H., Miyata, T., and Honjo, T., (1998), J Exp Med 188: 2151-2162.). With regard to light chain, the κ chain V_{K}3 of HO538-213 showed homology of 97% with the germ cell gene L6 (Huber, C., Schable, K.F., Huber, E., Klein, R., Meindl, A., Thiebe, R., Lamm, R., and Zachau, H.G., (1993), Eur J Immunol 23: 2868-2875.; Pech, M., and Zachau, H. G., (1984), Nucleic Acids Res 12: 9229-9236.), and the κ chains Vκ1 of HO702-001 and HO702-016 both showed homology of 97% with the germ cell gene L12 (Pech, M., and Zachau, H. G., (1984), Nucleic Acids Res 12: 9229-9236.; Bentley, D. L., and Rabbits, T. H., (1980), Nature 288: 730-733.). These data suggest that somatic hypermutation (sHM) occurred in these IgM variable region genes. Moreover, if taking into consideration the number of V_{H} and V_{L} genes encoded by the genome, it is also suggested that, apparently, the V_{H} and V_{L} genes are selectively used to generate a human CD4-reactive antibody.

The putative amino acid sequences of V_{H} and V_{L} are shown in Figure 3. The light chain of the clone HO702-001 was identical to that of the clone HO702-016. HO538-213 and HO702-001 are both derived from the germ cell gene V_{H} 3-33, but their V_{H} amino acid sequences are different. Thus, it is suggested that affinity maturation has occurred due to sHM.

Whether or not these human CD4-reactive Fab antibodies have influence on the replication of HIV-1 was examined. Virus replication was monitored by measuring a p24^{cA} viral antigen, which had been released by the primary cultured monocytes into the culture supernatant. Among the three IgM Fab clones, HO538-213 suppressed the replication of HIV-1_{JR-FL} at concentrations of 1 µg/ml and 2.5 µg/ml. However, there was observed no influence at a concentration of 0.2 µg/ml (Figure 4). In order to examine the epitope of human CD4, a competition assay was carried out between HO538-213 and anti-CD4 mouse monoclonal antibodies Leu-3a and RPA-T4 that neutralize HIV-1 (see Non Patent Documents 7 and 8, and Sattentau, Q. J., Dalgleish, A. G., Weiss, R. A., and Beverley, P. C., (1986), Science 234: 1120-1123.23). HO538-213 inhibited neither the binding of human CD4 with Leu-3a nor with RPA-T4. This result suggested that the epitope recognized by HO538-213 is different from the epitope recognized by the two types of monoclonal antibodies neutralizing HIV-1. It was demonstrated that Leu-3a inhibits the immunoregulatory function of human CD4⁺ T cells. On the other hand, HO538-213 can be anticipated to be a strong HIV-1 blocker that does not inhibit the original function of human CD4.

### 3. Consideration

The aforementioned results demonstrated that B cells from healthy adult peripheral blood encode IgM having sHM and exhibiting cross-reactivity with human CD4. IgM exhibits multireactivity with respect to pathogens and provides natural resistance to infectious pathogens. The aforementioned results show that human CD4-reactive IgM is present in a healthy body and is able to contribute to natural resistance to HIV-1 infection or the progression of AIDS, without causing abnormality in the functional regulation of human CD4⁺ T cells. This clearly shows the congenital function of IgM to an "unencountered" pathogen (which indicates HIV-1 herein) at a clone level.

Infants have humoral natural immunity mediated by IgM generated from IgM⁺IgD⁺CD27⁺ B cells. Thus, sHM against unencountered pathogens is accumulated in such infant's body. The aforementioned results show that a mature B cell population from adult peripheral blood also generates congenital IgM attended with sHM. If taking into consideration the fact that these IgM-generating B cells undergo positive and negative selection, the original target may not be human CD4. That is, there is a possibility that sHM is accumulated in an IgM Fab gene and that cross-reactivity with human CD4 may occur after B cell maturation in the peripheral blood. It is considered that a small amount of human CD4-cross-reactive IgM is produced *in vivo,* and that the subsets of these clones are able to function as innate defense to HIV-1 infection. B-1 cells generate such IgM and show cross-reactivity with an autoantigen in many cases. When such IgM causes abnormality in the functional regulation of human CD4⁺ T cells, the expression of CD5 is induced in antibody-generating B cells, and the generation of an autoreactive antibody is self-controlled by an unknown mechanism.

The aforementioned results clarify that V_{H} and V_{L} genes are selectively used to generate human CD4-reactive IgM Fab. This principle is considered to be true of other self-recognizing antibodies, and a strong anti-NIV-1 antibody having cross-reactivity with an autoantigen and showing broad neutralizing activity may be included in such other self-recognizing antibodies. In order to obtain findings regarding the development of an antibody-type AIDS vaccine, a human monoclonal antibody that neutralizes HIV-1 must be further isolated, and the sequence of the variable region thereof or cross-reactivity with an autoantigen must be analyzed. The above-described experimental procedures are likely to be useful for coping with these problems.

When a self-recognizing antibody is used as a therapeutic agent, safety always becomes an important issue. A donor, from which human CD4-reactive IgM Fab had been isolated as HO538-213, maintained good health for 20 years or more after the isolation. This suggests that such isolation did not seriously inhibit the function of human CD4⁺ T cells *in vivo.* A healthy subject-derived human CD4-reactive antibody is highly likely to be useful for the treatment of HIV-1 infection. If a cloned human CD4-reactive antibody has an immunoregulatory function, it can be anticipated that the antibody can be used for the treatment of transplant patients and patients with other immunological diseases.

### Industrial Applicability

According to a report from the Joint United Nations Programme on HIV/AIDS (UNAIDS), the number of people infected with HIV (including children) was assumed to be 33,000,000 (the percentage of adults infected with HIV: 0.8%) over the world at the end of 2007. HIV is a highly contagious virus, which is contained in a large amount in the blood, semen and vaginal discharge of the HIV-infected people, and which can be easily transmitted to uninfected people via sexual infection, blood infection, mother-to-child transmission, etc. Furthermore, it has been known that a person who is infected with HIV is highly likely to develop AIDS and to result in death. Under such circumstances, HIV infection has been treated as a social problem not only in Japan but also over the world. The agent for suppressing the replication of HIV and pharmaceutical agent of the present invention have many targets (people infected with HIV and patients affected with AIDS), to which they can be applied, and thus, the present invention is industrially highly effective. Further, the present invention is able to prevent the spread of HIV around the world, and thus it has a high social value.

## Claims

1. An agent for suppressing the replication of human immunodeficiency virus, which comprises, as an active ingredient, an antibody described in (1) below:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing.

2. An agent for suppressing the replication of human immunodeficiency virus, which comprises, as an active ingredient, a nucleic acid comprising nucleotide sequences encoding an antibody described in (1) below:
(1) an antibody recognizing human CD4, which comprises, as the amino acid sequence of the variable region of a heavy chain thereof, the amino acid sequence shown in SEQ ID NO: 2 in the sequence listing, or an amino acid sequence having at least one type of mutation selected from the group consisting of deletion, substitution, inversion, addition and insertion of one or multiple amino acids with respect to the amino acid sequence shown in SEQ ID NO: 2 in the sequence listing; and which also comprises, as the amino acid sequence of the variable region of a light chain thereof, the amino acid sequence shown in SEQ ID NO: 4 in the sequence listing, or an amino acid sequence having at least one type of mutation selected from the group consisting of deletion, substitution, inversion, addition and insertion of one or multiple amino acids with respect to the amino acid sequence shown in SEQ ID NO: 4 in the sequence listing.

3. The agent for suppressing the replication of human immunodeficiency virus according to claim 1 or 2, wherein the antibody recognizes an epitope present in a site of human CD4 that binds to gp120, which is different from the epitope recognized by Leu3a and/or RPA-T4.

4. The agent for suppressing the replication of human immunodeficiency virus according to any one of claims 1 to 3, wherein the antibody is a Fab antibody.

5. An agent for suppressing the replication of human immunodeficiency virus, which comprises, as an active ingredient, a nucleic acid described in (2) below:
(2) a nucleic acid comprising the nucleotide sequence shown in SEQ ID NO: 1 in the sequence listing and the nucleotide sequence shown in SEQ ID NO: 3 in the sequence listing.

6. The agent for suppressing the replication of human immunodeficiency virus according to any one of claims 2 to 5, wherein the nucleic acid comprised as an active ingredient is inserted into a vector.

7. The agent for suppressing the replication of human immunodeficiency virus according to claim 6, wherein the vector is a plasmid vector or a virus vector.

8. The agent for suppressing the replication of human immunodeficiency virus according to any one of claims 1 to 7, wherein the human immunodeficiency virus is human immunodeficiency virus type 1.

9. A pharmaceutical agent for preventing and/or treating acquired immune deficiency syndrome, which comprises, as an active ingredient, the agent for suppressing the replication of human immunodeficiency virus according to any one of claims 1 to 8.


SEQUENCE LISTING

<110>  Tokai University Educational System

<120>  HIV replication inhibitor, and use thereof

<130>  P16375EP

<140>  EP 10 735 603.2
<141>  2010-01-21

<150>  JP20090017340
<151>  2009-01-28

<160>  26

<170>  PatentIn version 3.3

<210>  1
<211>  378
<212>  DNA
<213>  Homo sapiens

<400>  1
caggtgcagc tggtgcagtc tgggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt gtctgctatg atggaagaac tacttactat     180

gcagactccg tgaagggccg attcaccatc tccagagata tttccgaaaa cacgctgtat     240

ttgcaaatga acagcctgag agccgaggac acggctgtat attactgtgc gaaagatctt     300

aataagggat attgtaataa tggtggctgc tacggggttg actgttgggg ccagggaacc     360

ctggtcaccg tctcctca                                                   378

<210>  2
<211>  126
<212>  PRT
<213>  Homo sapiens

<400>  2

Gln Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Val Cys Tyr Asp Gly Arg Thr Thr Tyr Tyr Ala Asp Ser Val
    50                  55                  60

Lys Gly Arg Phe Thr Ile Ser Arg Asp Ile Ser Glu Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Leu Asn Lys Gly Tyr Cys Asn Asn Gly Gly Cys Tyr Gly
            100                 105                 110

Val Asp Cys Trp Gly Gln Gly Thr Leu Val Thr Val Ser Ser
        115                 120                 125

<210>  3
<211>  327
<212>  DNA
<213>  Homo sapiens

<400>  3
gaaattgtga tgacgcagtc tccagccacc ctgtctttgt ctccagggga aagagccacc      60

ctctcctgca gggccagtca gagtgttaac aactacttag cctggtacca acagagacct     120

ggccaggctc ccagcctcct catctatgat gcatccaaca gggccactgg catcccagcc     180

aggttcagtg gcagtgggtc tgggacagac ttcactctca ccatcagcag cctagagcct     240

gaagattttg cagtttatta ctgtcagcaa cgtggcaact ggcctcatac ttttggccag     300

gggaccaagc tggagatcaa acgaact                                         327

<210>  4
<211>  109
<212>  PRT
<213>  Homo sapiens

<400>  4

Glu Ile Val Met Thr Gln Ser Pro Ala Thr Leu Ser Leu Ser Pro Gly
1               5                   10                  15

Glu Arg Ala Thr Leu Ser Cys Arg Ala Ser Gln Ser Val Asn Asn Tyr
            20                  25                  30

Leu Ala Trp Tyr Gln Gln Arg Pro Gly Gln Ala Pro Ser Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Gly Asn Trp Pro His
                85                  90                  95

Thr Phe Gly Gln Gly Thr Lys Leu Glu Ile Lys Arg Thr
            100                 105

<210>  5
<211>  44
<212>  DNA
<213>  Artificial

<220>
<223>  synthetic DNA

<400>  5
aaggcccaac cggccatggc ccaggtgcag ctggtgcagt ctgg                    44


<210>  6
<211>  44
<212>  DNA
<213>  Artificial

<220>
<223>  synthetic DNA

<400>  6
aaggcccaac cggccatggc ccagrtycag ctggtgcagt ctgg                    44


<210>  7
<211>  44
<212>  DNA
<213>  Artificial

<220>
<223>  synthetic DNA

<400>  7
aaggcccaac cggccatggc ccagstrcag ctgcagsagt crgg                    44


<210>  8
<211>  44
<212>  DNA
<213>  Artificial

<220>
<223>  synthetic DNA

<400>  8
aaggcccaac cggccatggc csagrtgcar ctgktggagt ctgg                    44


<210>  9
<211>  44
<212>  DNA
<213>  Artificial

<220>
<223>  synthetic DNA

<400>  9
aaggcccaac cggccatggc ccaggtgcag ctacagsagt gggg                    44


<210>  10
<211>  34
<212>  DNA

<213>  Artificial

<220>
<223>  synthetic DNA

<400>  10
ccgcggccgc tgtgtgagtt ttgtcacaag attt                                34


<210>  11
<211>  34
<212>  DNA
<213>  Artificial

<220>
<223>  synthetic DNA

<400>  11
ccgcggccgc tttgcgctca actgtcttgt ccac                                34


<210>  12
<211>  34
<212>  DNA
<213>  Artificial

<220>
<223>  synthetic DNA

<400>  12
ccgcggccgc tgtgtgagtt gtgtcaccaa gtgg                                34


<210>  13
<211>  34
<212>  DNA
<213>  Artificial

<220>
<223>  synthetic DNA

<400>  13
ccgcggccgc tgggggacca tatttggact caac                                34


<210>  14
<211>  34
<212>  DNA
<213>  Artificial

<220>
<223>  synthetic DNA

<400>  14
ccgcggccgc cactggaaga ggcacgttct tttc                                34


<210>  15
<211>  31
<212>  DNA
<213>  Artificial

<220>
<223>  synthetic DNA

<400>  15
ccgctagcgm catycagwtg acccagtctc c                                    31


<210>  16
<211>  31
<212>  DNA
<213>  Artificial

<220>
<223>  synthetic DNA

<400>  16
ccgctagcga trttgtgatg acycagwctc c                                    31


<210>  17
<211>  31
<212>  DNA
<213>  Artificial

<220>
<223>  synthetic DNA

<400>  17
ccgctagcga aattgtgwtg acgcagtctc c                                    31


<210>  18
<211>  31
<212>  DNA
<213>  Artificial

<220>
<223>  synthetic DNA

<400>  18
ccgctagcga catcgwghtg acccagtctc c                                    31


<210>  19
<211>  34
<212>  DNA
<213>  Artificial

<220>
<223>  synthetic DNA

<400>  19
ttggcgcgcc acactctccc ctgttgaagc tctt                                 34


<210>  20
<211>  29
<212>  DNA
<213>  Artificial

<220>
<223>  synthetic DNA

<400>  20
ccgctagcca gtctgysctg actcagccw                                       29


<210>  21

<211>  29
<212>  DNA
<213>  Artificial

<220>
<223>  synthetic DNA

<400>  21
ccgctagcca gtctgtgytg acgcagccg                                          29


<210>  22
<211>  29
<212>  DNA
<213>  Artificial

<220>
<223>  synthetic DNA

<400>  22
ccgctagcma ckttataytg actcaaccg                                          29


<210>  23
<211>  29
<212>  DNA
<213>  Artificial

<220>
<223>  synthetic DNA

<400>  23
ccgctagcca gactgtggta acycaggag                                          29


<210>  24
<211>  29
<212>  DNA
<213>  Artificial

<220>
<223>  synthetic DNA

<400>  24
ccgctagctc ctatgwgctg actcagcca                                          29


<210>  25
<211>  29
<212>  DNA
<213>  Artificial

<220>
<223>  synthetic DNA

<400>  25
ccgctagctc ttctgagctg actcaggac                                          29


<210>  26
<211>  34
<212>  DNA
<213>  Artificial

<220>

<223>  synthetic DNA

<400>  26
ttggcgcgcc tgaamatkct gtagsggcca ctgt                                    34